# EUROPEAN PATENT SPECIFICATION

(11) **EP 0 996 437 B1**
(45) Date of publication and mention of the grant of the patent: **16.05.2001**
(21) Application number: 98937444.2
(22) Date of filing: 04.06.1998
(51) Int. Cl.: A61K 31/192, A61K 47/18

(54) **SEMISOLID PHARMACEUTICAL FORMULATION CONTAINING DEXKETOPROFEN TROMETAMOL**
HALBFESTE PHARMAZEUTISCHE ZUBEREITUNGEN ENTHALTEND DEXKETOPROFEN-TROMETAMOL
FORMULATION PHARMACEUTIQUE SEMI-SOLIDE CONTENANT DU DEXKETOPROFENE TROMETAMOL

(30) Priority: 04.06.1997 ES 9701213
(43) Date of publication of application: 03.05.2000
(73) Proprietor: LABORATORIOS MENARINI S.A., 08912 Badalona (ES)
(72) Inventor: MAULEON CASELLAS, David, E-08191 Rubi (ES); PALOMER BENET, Albert, E-17002 Girona (ES); GARCIA PEREZ, Luisa, 08340 Villassar de Mar (ES)
(74) Representative: Minoja, Fabrizio, Dr.
(86) International application number: EP9803333
(87) International publication number: WO9855113

(56) References cited:
- WO-A-94/11332
- BE-A- 886 486
- ES-A- 538 994
- US-A- 5 091 182

## Description

### FIELD OF THE INVENTION

The present invention relates to a process for the preparation of a semisolid pharmaceutical formulation (gel or cream) containing dexketoprofen trometamol and different amines as neutralizing agents, for the topical treatment of inflammatory processes in humans. The pharmaceutical formulations obtained by said process are also object of the present invention and show advantages deriving from their high storage stability without noticeable changes in the appearance of the product in time, a well as from their lower allergenic and photoallergenic activities.

### DESCRIPTION OF THE PRIOR ART

The use of semisolid formulations (gels or creams) of non steroidal antiinflammatory agents (NSAIDS) is common in the topical treatment of inflammatory syndromes.

Most NSAID used in therapy, for example arylpropionic or arylacetic acids derivatives, have an acid function in their structure. Carboxyvinyl polymers (carbomer or carbopol^{(R)}) can be used as gelifying agents in the preparation of gels with this type of NSAID, these polymers need the mandatory presence of a neutralizing base to increase their viscosity and to attain their correct gelification. In order to obtain a good gelification with this type of compounds, the base has to neutralize both the gelifying agent and the active principle. The most widely used bases for this purpose are amines, such as triethanolamine and diethanolamine. Recently, tromethamine [DCI trometamol, 2-amino-2-(hydroxyethyl)-1,3-propanediol] has also been used as neutralizing agent in the preparation of gels for therapeutic use, due to its suitable physical-chemical characteristics as well as to its low toxicity (Bremecker K.D. Pharm. Ind. 51 (2) 199-202 (1989)).

Tromethamine has remarkable advantages both from the physical-chemical and from the toxicologic point of view, compared with the commonly used amines, such as triethanolamine and diethanolamine. As far as its physical-chemical characteristics are concerned, tromethamine is a weakly basic polar amine (pK_{b} = 7.8) which forms buffer solutions of about the plasma pH, therefore it is also used as isotonic solution (Martindale 31st Ed.) and to control the body and blood pH during transfusions. From the toxicologic point of view, triethanolamine and diethanolamine proved to be potentially harmful, in particular carcinogenic, compounds both in *in vitro* cellular models (Santamaria A. et al. Ecotoxicol. Environ. Saf. 34 (1) 56-58 (1996)) and *in vivo* in rodents (Konishi Y. et al. Fundament. Appl. Toxicol. 18 (1) 25-29 (1992); Melnick R. L. et al. J. Appl. Toxicol. 14 (1) 1-9 (1994) and ibid. 14 (1) 1-9 (1994)), by oral as well as by topical administration. On the other hand, when included in creams and gels, triethanolamine caused irritating reactions resulting from its combination with other components of the formulation, e.g. stearates. These compounds showed a powerfully irritating action in humans (Batten T. L. et al. Contact Dermatitis 30, 159-161 (1994)). In its turn, tromethamine reduces or removes any risk of formation of carcinogenic nitrosoamines (Bremecker K.D. Pharm. Ind. 51 (2) 199-202 (1989)), in that, being it a primary amine, it forms unstable nitrosoamines, which decompose in the presence of the nitrosating agent. Moreover, tromethamine itself is used as a medicament in respiratory and metabolic acidosis syndromes (USP XXIII and European Pharmacopoeia 3rd Ed.).

In literature, the preparation of pharmaceutical formulations containing tromethamine for the oral administration of non steroidal antiinflammatory compounds has been described. In these formulations, the therapeutical agent, for example diflunisal, is mixed with tromethamine and the required adjuvants (Phillips A. EP 293529, 1988), or the salt of the therapeutical agent is used, e.g. dexketoprofen trometamol (Carganico G. et al. IS 9202260, 1992), which improves the oral absorption rate of the medicament as well as the onset of its analgesic action.

As mentioned above, tromethamine has recently been used also as neutralizing agent in the preparation of gels for the therapeutic use. The physical characteristics of the resulting pharmaceutical formulations are similar to those obtained with triethanolamine, as far as pH, viscosity, organoleptic characteristics and storage stability are concerned (Kim H. et al. JP 07309938, 1995 and Tamburick S. et al. Pharm. Res. 13 (2) 279-83 (1996)). Moreover, the use of tromethamine as neutralizing agent allows, due to its lower basicity, to add the amine in larger amounts than the stoichiometric and, as a consequence, the maximum viscosity area does not take place after the stoichiometric addition of the amine, as it happens when using triethanolamine or diethanolamine, but after the addition of larger amounts of the amine, the buffering power of the resulting formulation being much higher (Bremecker K.D. Pharm. Ind, 51 (2) 199-202 (1989)). Tromethamine has been used in pharmaceutical semisolid formulations (gels and liposomes) for the treatment of acne and psoriasis (Charu P.M. WO 9533489, 1995 and Hager J.-C. DE 4322158, 1995), in oxytocic gels containing prostaglandins E, A and F2a (Kravciv M. et al. CS 276067, 1992 and CS 277720, 1993)) and, together with salicylic and nicotinic acids, in ointments for the analgesic topical treatment of muscular pain of rheumatic and arthritic origin or post- athletic training pains (GB 1106580, 1968). Finally, semisolid pharmaceutical formulations have been described containing ketorolac tromethamine salt for the administration of this compound in the form of dentifrice gel (Cavanaugh P.F. et al. WO-9628144 A1, 1996) or of solution for mouth rinse (Cavanaugh P.F. et al. inflammopharmacology 3(4) 313 (1995) and Kelm G.R. et al. J. Pharm. Sci. 85 (8) 842 (1996)) in the treatment of periodontitis and other buccal diseases.

The present invention aims therefore at obtaining gels of the analgesic and antiinflammatory medicament dexketoprofen trometamol having a complete storage stability without noticeable changes in the appearance of the product in time.

According to the prior art, a polyacrylic gel of an arylpropionic acid can be prepared starting from the active principle as the free acid, dissolving it in a suitable solvent such as ethanol and adding it with a dispersion formed by carbomer and water; finally, neutralizing it with a physiologically compatible amine, such as triethanolamine, diethanolamine or tromethamine, to obtain the desired gel. The dexketoprofen gel (at a concentration ranging between 1 and 3%) prepared according to this method, using triethanolamine or diethanolamine as the amine, tends to become muddy after 1-6 months due to the precipitation of the active principle as such or as a salt with the neutralizing amine. This precipitation is even more evident at temperatures below 20°C, which are often those corresponding to the usual storage conditions.

According to the present invention, particurarly using tromethamine as neutralizing agent, no precipitation occurs, thereby obtaining a gel with a high stability (higher than 2 years) and with favourable rheological and thixotropic characteristics.

Surprisingly, when this type of gels is prepared using dexketoprofen trometamol as active principle instead of dexketoprofen and the amine in just the necessary amount to neutralize the gelifying agent, no precipitation with any of the used amines is observed. A great variety of amines can be used as neutralizing agents, such as triethanolamine, diethanolamine, tromethamine, particularly preferred and convenient being the use of tromethamine also as neutralizing agent, in view of homogeneity with the amine salifying the active principle and, above all, of the lower toxicity exerted.

The dexketoprofen trometamol gel prepared with tromethamine turned out to be remarkably less photoallergenic than conventional gels.

The use of dexketoprofen trometamol as therapeutical agent together with diethanolamine or triethanolamine may be considered when a reduction in the amine content in the gel is desired.

The process for the preparation of a gel containing dexketoprofen trometamol at a concentration ranging between 1.5 and 4.5% (corresponding to 1-3% of dexketoprofen), typically comprises the formation first of a dispersion containing 0.5 to 5% of carbomer, 10-40% of ethanol and 60-90% of demineralized water, optionally in the presence of a suitable essence oil (0.05-0.5%). This dispersion is then added with a solution containing dexketoprofen trometamol (from 1.5 to 4.5% on the total amount of gel) and a suitable amine (from 0.5 to 3% on the total amount of gel), such as tromethamine, triethanolamine or diethanolamine, in demineralized water (4-10% on the basis of the total amount of gel). After that, once the correct gelification takes place, pH is checked to range between 5 and 7, preferably between 6 and 6.5. Finally, the gel is de-aerated under mild vacuum.

When tromethamine is used as neutralizing agent, dexketoprofen trometamol can also be prepared in situ. In this case, the process consists in the preparation of a 1-3% dexketoprofen solution on the basis of the total amount of gel in ethanol (25-40%) containing a suitable essence oil (0.05-0.5%), which solution is then added with a dispersion of 0.5-5% of carbomer in water. After a suitable time from 15 to 30 min. for the correct homogenization of the formed dispersion, tromethamine is added in an equimolar amount to dexketoprofen (between 0.5 and 1.5%), and finally carbomer is neutralized with 0.5-3% of a physiologically compatible amine, such as triethanolamine, diethanolamine or tromethamine, to adjust pH to 5 - 7, preferably to 6 - 6.5. Finally, the gel is de-aerated under mild vacuum.

The gels of the invention have a long-term storage stability under conventional conditions (15-35°C) for long periods of time and exhibit excellent antiinflammatory and analgesic effects without involving noticeable toxic effects when directly applied to the skin.

### EXAMPLES

The following examples further illustrate the process of the invention.
Example 1: Preparation of a gel starting from tromethamine (+)-(S)-2-(3-benzoylphenyl)propionate using tromethamine as neutralizing agent.

A 200 L reactor equipped with a stirrer is loaded with 40.8 L of ethanol, 0.1 kg of a suitable essence oil, 57.5 L of demineralized water and 1.5 kg of carbomer. The mixture is stirred to a homogeneous dispersion (15 min). The resulting dispersion is added with a solution of 1.4 kg of tromethamine, 1.85 kg of tromethamine (+)-(S)-2-(3-benzoylphenyl)propionate and 5.65 L of water, stirring to obtain a correct incorporation (15 min). After this time, stirring is stopped and pH is checked to range from 6 to 6.5. Finally, the resulting gel is de-aerated under mild vacuum.
Example 2: Preparation of a gel starting from tromethamine (+)-(S)-2-(3-benzoylphenyl)propionate using diethanolamine as neutralizing agent.

A 200 L reactor equipped with a stirrer is loaded with 40.8 L of ethanol, 0.1 kg of a suitable essence oil, 57.5 L of demineralized water and 1.5 kg of carbomer. The mixture is stirred to a homogeneous dispersion (15 min). The resulting dispersion is added with a solution of 1.04 kg of diethanolamine, 1.85 kg of tromethamine (+)-(S)-2-(3-benzoylphenyl)propionate and 6.01 L of water, stirring to obtain a correct incorporation (15 min). After this time, stirring is stopped and pH is checked to range from 6 to 6.5. Finally, the resulting gel is de-aerated under mild vacuum.
Example 3: Preparation of a gel starting from tromethamine (+)-(S)-2-(3-benzoylphenyl)propionate using triethanolamine as neutralizing agent.

A 200 L reactor equipped with a stirrer is loaded with 40.8 L of ethanol, 0.1 kg of a suitable essence oil, 57.5 L of demineralized water and 1.5 kg of carbomer. The mixture is stirred to a homogeneous dispersion (15 min). The resulting dispersion is added with a solution of 1.58 kg of triethanolamine, 1.85 kg of tromethamine (+)-(S)-2-(3-benzoylphenyl)propionate and 5.47 L of water, stirring to obtain a correct incorporation (15 min). After this time, stirring is stopped and pH is checked to range from 6 to 6.5. Finally, the resulting gel is de-aerated under mild vacuum.
Example 4: Preparation of a gel starting from (+)-(S)-2-(3-benzoylphenyl)propionic acid.

A 1500 L reactor equipped with a stirrer is loaded with 408 litres of ethanol and 12.5 kg of (+)-(S)-2-(3-benzoylphenyl)propionic acid and 1.0 kg of a suitable essence oil. The mixture is stirred for 15 minutes. After this time, it is added with a dispersion of carbomer (15 kg) and demineralized water (575 L), prepared mixing the two components and stirring vigorously by a mechanical stirrer. The resulting dispersion is stirred until a correct homogenization (20 mini, subsequently it is added with a solution of tromethamine (21 kg) in demineralized water (55.5 L) and stirred to obtain a correct incorporation (15 min). After this time, stirring is stopped and pH is checked to range from 6 to 6.5. Finally, the resulting gel is de-aerated under mild vacuum.

## Claims

1. A process for the preparation of a semisolid pharmaceutical formulation containing (+)-(S)-2-(3-benzoylphenyl)propionic acid tromethamine salt (dexketoprofen trometamol) as therapeutical agent, a gelifying agent and a pharmacologically acceptable amine as neutralizing agent, which process comprises the addition of a solution of the neutralizing amine and of dexketoprofen tromethamol to a dispersion of the gelifying agent in a suitable solvent.

2. A process according to claim 1, wherein the gelifying agent is a carboxyvinyl polymer.

3. A process according to claim 1, wherein the neutralizing agent is selected from diethanolamine, triethanolamine and tromethamine.

4. A process according to claim 3, wherein the neutralizing agent is tromethamine.

5. A process according to any one of claims 1-4, wherein the pH is adjusted ranging from 5 to 7.

6. A process according to claim 5, wherein the pH is adjusted ranging from 6 to 6.5.

7. A process according to any one of claims 1-4, wherein the semisolid formulations is a gel or cream.

8. A semisolid pharmaceutical formulation of dexketoprofen tromethamol comprising a gelifying agent and a neutralizing agent selected from diethanolamine, triethanolamine and tromethamine.

9. A semisolid pharmaceutical formulation according to claim 8 wherein the neutralizing agent is tromethamine.

10. A semisolid pharmaceutical formulation according to claim 8 wherein the gelifying agent is a carboxyvinyl polymer.

## Patentansprüche

1. Ein Verfahren zur Herstellung einer halbfesten pharmazeutischen Formulierung, die (+)-(S)-2-(3-Benzoylphenyl)propionsäure-Tromethaminsalz (Dexketoprofen-Tromethamol) als therapeutisches Mittel, ein Geliermittel und ein pharmazeutisch verträgliches Amin als Neutralisationsmittel enthält, wobei das Verfahren das Zugeben einer Lösung des neutralisierenden Amins und Dexketoprofen-Tromethamol zu einer Dispersion des Geliermittels in einem geeigneten Lösungsmittel umfasst.

2. Verfahren nach Anspruch 1, wobei das Geliermittel ein Carboxyvinylpolymer ist.

3. Verfahren nach Anspruch 1, wobei das Neutralisationsmittel aus Diethanolamin, Triethanolamin und Tromethamin ausgewählt ist.

4. Verfahren nach Anspruch 3, wobei das Neutralisationsmittel Tromethamin ist.

5. Verfahren nach einem der Ansprüche 1 bis 4, wobei der pH-Wert im Bereich von 5 bis 7 eingestellt wird.

6. Verfahren nach Anspruch 5, wobei der pH-Wert im Bereich von 6 bis 6,5 eingestellt wird.

7. Verfahren nach einem der Ansprüche 1 bis 4, wobei die halbfeste Formulierung ein Gel oder eine Creme ist.

8. Eine halbfeste pharmazeutische Formulierung von Dexketoprofen-Tromethamol, die ein Geliermittel und ein Neutralisationsmittel ausgewählt aus Diethanolamin, Triethanolamin und Tromethamin umfasst.

9. Eine halbfeste pharmazeutische Formulierung nach Anspruch 8, wobei das Neutralisationsmittel Tromethamin ist.

10. Eine halbfeste pharmazeutische Formulierung nach Anspruch 8, wobei das Geliermittel ein Carboxyvinylpolymer ist.

## Revendications

1. Procédé pour la préparation d'une composition pharmaceutique semi-solide contenant le sel de trométhamine de l'acide (+)-(S)-2-(3-benzoylphényl)propionique (dexcétoprofène trométamol) en tant qu'agent thérapeutique, un agent gélifiant et une amine pharmacologiquement acceptable en tant qu'agent neutralisant, procédé qui comprend l'addition d'une solution de l'amine neutralisante et du dexcétoprofène trométhamol à une dispersion de l'agent gélifiant dans un solvant approprié.

2. Procédé selon la revendication 1, dans lequel l'agent gélifiant est un polymère carboxyvinylique.

3. Procédé selon la revendication 1, dans lequel l'agent neutralisant est choisi parmi la diéthanolamine, la triéthanolamine et la trométhamine.

4. Procédé selon la revendication 3, dans lequel l'agent neutralisant est la trométhamine.

5. Procédé selon l'une des revendications 1 - 4, dans lequel le pH est ajusté dans la plage de 5 à 7.

6. Procédé selon la revendication 5, dans lequel le pH est ajusté dans la plage de 6 à 6,5.

7. Procédé selon l'une des revendications 1 - 4, dans lequel la composition semi-solide est un gel ou une crème.

8. Composition pharmaceutique semi-solide de dexcétoprofène trométhamol comprenant un agent gélifiant et un agent neutralisant choisi parmi la diéthanolamine, la triéthanolamine et la trométhamine.

9. Composition pharmaceutique semi-solide selon la revendication 8, dans laquelle l'agent neutralisant est la trométhamine.

10. Composition pharmaceutique semi-solide selon la revendication 8, dans laquelle l'agent gélifiant est un polymère carboxyvinylique.
